# EUROPEAN PATENT SPECIFICATION

(11) **EP 1 507 572 B1**
(45) Date of publication and mention of the grant of the patent: **10.01.2007**
(21) Application number: 03731304.6
(22) Date of filing: 16.05.2003
(51) Int. Cl.: A61M 37/00, A61M 35/00, A61B 17/00, B05C 17/005

(54) **Wound closure material applicator**
Produktapplicator zum Schliessen von Wunden
Applicateur de matière de fermeture de plaie

(30) Priority: 17.05.2002 US 381435 P
(43) Date of publication of application: 23.02.2005
(73) Proprietor: Tyco Healthcare Group LP, Norwalk, Connecticut 06856 (US)
(72) Inventor: CRISCUOLO, Christohper, J., Branford, CT 06405 (US); ARANYI, Ernie, Easton, CT 06612 (US)
(74) Representative: HOFFMANN EITLE
(86) International application number: PCT/US2003/016064
(87) International publication number: WO 2003/097157

(56) References cited:
- WO-A-95/08951
- WO-A-99/02207
- US-A- 4 033 484
- US-A- 5 501 666
- US-A- 5 645 886
- US-B1- 6 170 714
- US-B1- 6 224 567

## Description

### BACKGROUND:

### 1. Technical Field

The present disclosure relates to an applicator for applying a fluid material onto a surface. More specifically, the present disclosure relates to an applicator for applying a wound closure material onto tissue for wound repair. The technical features of the pre-characterizing part of claim 1 are disclosed in WO 95/08951-A.

### 2. Background of Related Art

Applicators for applying wound closure materials, for example, adhesives, sealants and hemostats, to tissue to effect wound repair are well known in the art. Typically, such applicators include a tube or syringe and a plunger or driver which is advanceable through the syringe to force the wound closure material from the syringe. Such a device is disclosed in U.S. Patent No. 6,027,471. In such devices, the distal end of the syringe is open and the wound closure material is retained within the syringe by surface adhesion of the wound closure material to the internal walls of the syringe. After the wound closure material is dispensed from the syringe, drippage of the wound closure material from the distal end of the syringe is common. Due to the location of use of wound closure material applicators, e.g., body cavities, body tissue, etc., such drippage is undesirable.

Accordingly, a need exists for a wound closure material applicator which is easy to operate, minimizes drippage and provides a more controlled application of the material to tissue.

### SUMMARY

In accordance with one preferred embodiment of the present disclosure, a wound closure material applicator is provided which includes a handle assembly having a movable handle or trigger and a stationary handle. A body portion extends distally from the handle assembly and defines a body channel dimensioned to receive wound closure material or a reservoir of wound closure material. A tip assembly is supported adjacent the distal end of the body portion and includes a seal member, a valve seat and a discharge channel positioned distally of the valve seat. The seal member is movable distally within the tip assembly from a closed position in which the seal member engages the valve seat to an open position in which the seal member is spaced from the valve seat. The tip assembly is configured such that movement of the seal member from the open position to the closed position causes a reduction of pressure within the discharge channel of the tip assembly to minimize drippage of wound closure material from the applicator. Preferably, the seal member is urged toward the closed position by a biasing member.

Preferably, the tip assembly also includes a housing which defines an inlet channel and the valve seat, the valve seat being positioned adjacent the inlet channel. The seal member may include a spherical valve member positioned to engage the valve seat which can be formed integrally with or separately from the seal member. The seal member can also be configured to define the discharge channel. The tip assembly can also include a tip member having a concave or spherical receptacle which communicates with the discharge channel. One or a plurality of inlet ports may be formed in the seal member to communicate with the discharge channel. Preferably, the seal member includes an annular resilient finger which is positioned to engage a wall defining a bore in the tip assembly housing and provides a seal between the bore and the seal member. A feed bar can be provided within the body portion which is operatively associated with the movable handle such that actuation of the movable handle effects distal movement of the feed bar within the body portion. A pusher may be supported or integrally formed on a distal end of the feed bar. In a preferred embodiment, a drive plate provided in the handle assembly is driven into engagement with the feed bar by the movable handle to move the feed bar distally. A movable locking plate may also be provided in the handle assembly for permitting movement of the feed bar in a first position and for engaging and preventing proximal movement of the feed bar in a second position. Preferably, the locking member is urged to the second position by a biasing member.

In another preferred embodiment of the present disclosure, the tip assembly includes a housing, a tip member, and a permeable membrane. The permeable membrane is supported over the open end of a tip housing bore. In use, wound closure material is forced into the bore of the tip housing and passes through the permeable membrane into a spherical recess formed in the tip member. The presently disclosed wound closure material applicators provide a more controlled application of wound closure material onto a surface by minimizing dripping of the material from the applicator.

The wound closure material applicator can be used to dispense wound closure materials including adhesives, sealants and hemostats. One preferred adhesive is a cyanoacrylate adhesive.

### BRIEF DESCRIPTION OF THE DRAWINGS

Various preferred embodiments of the presently disclosed wound closure material applicator are described herein with reference to the drawings wherein:
FIG. 1 is a top view of one preferred embodiment of the presently disclosed wound closure material applicator;
FIG. 2 is a side view of the wound closure material applicator shown in FIG. 1;
FIG. 3 is a side view of the proximal portion of the wound closure material applicator shown in FIG. 1 including the handle assembly with a body half-section removed;
FIG. 4 is a front view of the drive plate of the wound closure material applicator shown in FIG. 1;
FIG. 5 is a side cross-sectional view of the drive plate shown in FIG. 4;
FIG. 6 is a side view of a feed bar guide member of the wound closure material applicator shown in FIG. 3;
FIG. 7 is a front view of the feed bar guide member shown in FIG. 6;
FIG. 8 is a side partial cross-sectional view of the guide member shown in FIG. 6;
FIG. 9 is a side view of the feed bar of the wound closure material applicator shown in FIG. 1;
FIG. 10 is a cross-sectional view taken along section lines 10-10 of FIG. 9;
FIG. 11 is a front view of the locking member of the wound closure material applicator shown in FIG. 1;
FIG. 12 is a cross-sectional view taken along section lines 12-12 of FIG. 11;
FIG. 13 is a cross-sectional cutaway view of the distal end of the locking member of the wound closure material applicator shown in FIG. 1;
FIG. 14 is a top view of the distal portion of the wound closure material applicator shown in FIG. with the outer tube removed;
FIG. 15 is a side view of the distal portion of the wound closure material applicator shown in FIG. 14;
FIG. 15A is a side cross-sectional view of the central portion of the wound closure material applicator shown in FIG. 1 with the pusher in a retracted position;
FIG. 15B is a side cross-sectional view of the distal portion of the wound closure material applicator shown in FIG. 1 with the pusher in an advanced position;
FIG. 16 is a top view with portions broken away of the outer tube of the wound closure material applicator shown in FIG. 1;
FIG. 17 is a top view of the inner tube of the wound closure material applicator shown in FIG. 1;
FIG. 18 is a side view of the inner tube shown in FIG. 17;
FIG. 18a is a cross-sectional view taken along section lines 18a-18a of FIG. 18;
FIG. 19 is a front view of the inner tube shown in FIG. 17;
FIG. 20 is a side cross-sectional view of the tip assembly of the wound closure material applicator shown in FIG. 1;
FIG. 20A is a side perspective view from the rear of the seal member of the tip assembly shown in FIG. 20;
FIG. 20B is a cross-sectional cutaway view of the proximal end of the seal member shown in FIG. 20a;
FIG. 21 is a side view of another preferred embodiment of the presently disclosed wound closure material applicator with a handle assembly body half-section removed;
FIG. 22 is a top view of the wound closure material applicator shown in FIG. 21;
FIG. 23 is a side view of the proximal portion of the wound closure material applicator shown in FIG. 21 including the handle assembly with a body half-section removed;
FIG. 24 is a side cross-sectional view of the wound closure material applicator shown in FIG. 21 with the body half-sections of the handle assembly removed;
FIG. 25 is a side view with portions broken away of the feed bar of the wound closure material applicator shown in FIG. 21;
FIG. 26 is a cross-sectional view taken along section lines 26-26 of FIG. 25;
FIG. 27 is a side view with portions broken away of the pusher of the wound closure material applicator shown in FIG. 21;
FIG. 28 is a top view with portions broken away of the pusher shown in FIG. 27;
FIG. 29 is a side perspective view of the piston of the wound closure material applicator shown in FIG. 21;
FIG. 30 is a side partial cross-sectional view of the piston shown in FIG. 29;
FIG. 31 is a side cross-sectional view of a distal portion of the wound closure material applicator shown in FIG. 21;
FIG. 31A is a side cross-sectional view of the tip assembly shown in FIG. 31 supported in the inner and outer tubes;
FIG. 32 is a side perspective view of the housing of the tip assembly shown in FIG. 31;
FIG. 33 is a side cross-sectional view of the housing shown in FIG. 32;
FIG. 34 is a side perspective view of the tip member of the tip assembly shown in FIG. 31;
FIG. 35 is a side cross-sectional view of the tip member shown in FIG. 34;
FIG. 36 is a side perspective view of the seal member of the tip assembly shown in FIG. 31;
FIG. 37 is a side cross-sectional view of the seal member shown FIG. 36;
FIG. 38 is a side cross-sectional view of the proximal end of the seal member shown in FIG. 36;
FIG. 39 is a side cross-sectional view with portions broken away of the distal portion of another preferred embodiment of the presently disclosed wound closure material applicator;
FIG. 40 is an enlarged cross-sectional view with portions broken away of the encircled distal portion of the tip assembly of the wound closure material applicator shown in FIG. 39;
FIG. 41 is a side perspective view of the tip member of another preferred embodiment of the presently disclosed wound closure material applicator;
FIG. 42 is a side partial cross-sectional view of the tip assembly shown in FIG. 42; and
FIG. 43 is a side cross-sectional view of the tip member of another preferred embodiment of the wound closure material applicator.

### DETAILED DESCRIPTION OF PREFERRED EMBODIMENTS

Preferred embodiments of the presently disclosed wound closure material applicator will now be described in detail with reference to the drawings, wherein like reference numerals designate identical or corresponding elements in each of the several views:

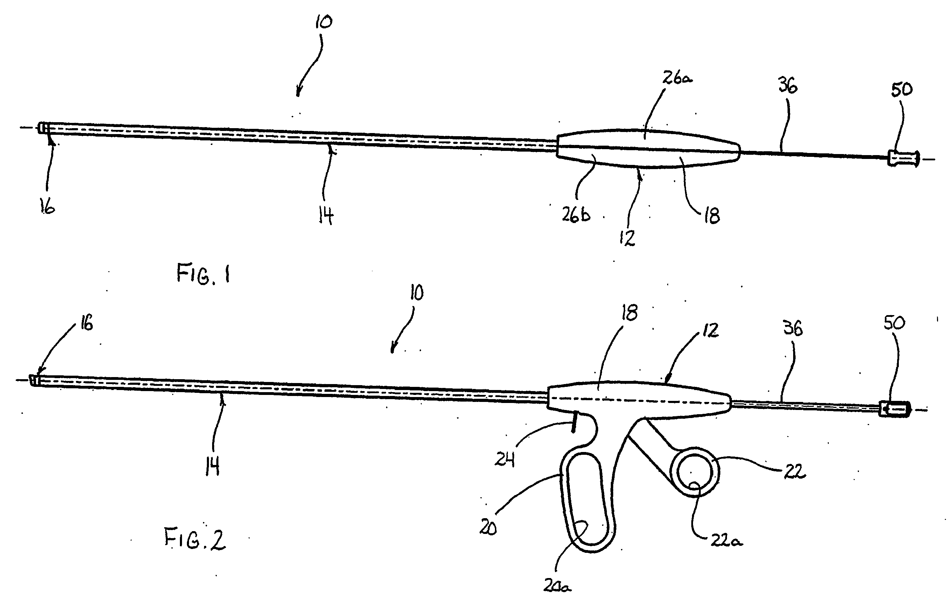

FIGS. 1 and 2 illustrate one embodiment of the presently disclosed wound closure material applicator shown generally as 10. Wound closure material applicator 10 includes a handle assembly 12, an elongated body portion 14 and a distally positioned tip assembly 16. Handle assembly 12 includes a barrel portion 18, a stationary handle portion 20, a movable handle portion or trigger 22 and a locking member 24. Handle portions 20 and 22 include finger loops 20a and 22a, respectively, which facilitate gripping of the instrument. Body portion 14 has a proximal end secured to handle assembly 12 and a distal end supporting tip assembly 16.

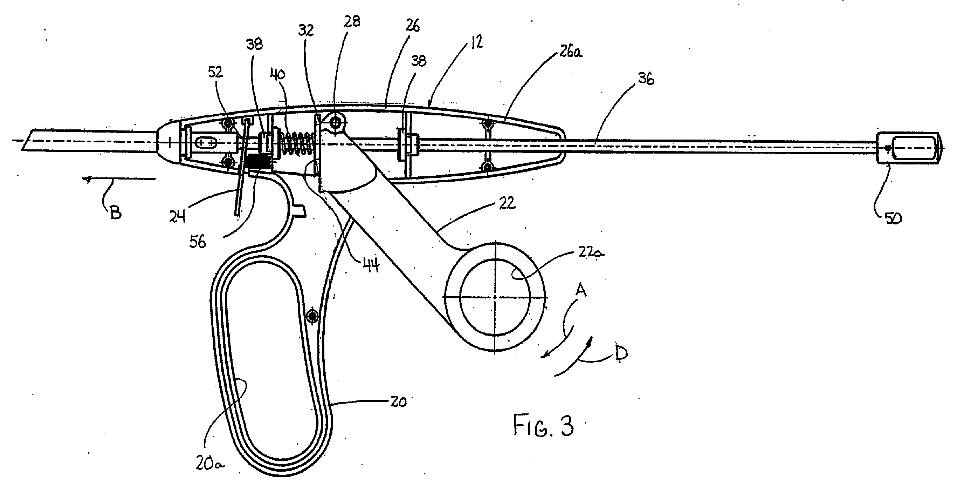

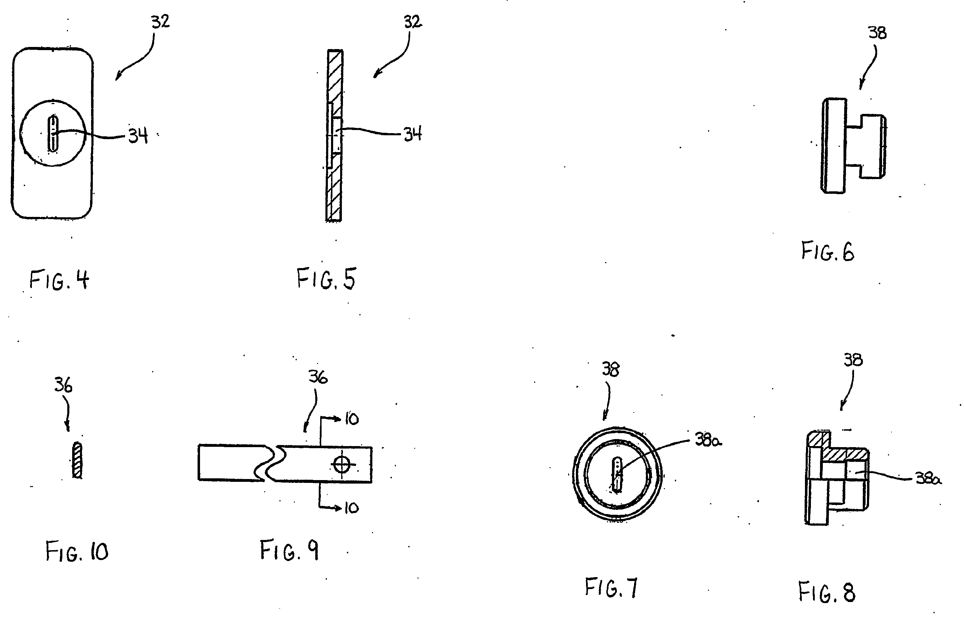

Referring to FIG. 3, handle assembly 12 includes a body 26 formed from body half-sections 26a and 26b (FIG. 1), which are preferably molded from a polycarbonate or other plastic material. Alternately, other materials may be used to form body half-sections 26a and 26b, e.g., stainless steel, etc. Trigger 22 is pivotally supported about a pivot member 28 which is supported between body half-sections 26a and 26b. Trigger 22 is pivotal in the direction indicated by arrow "A" towards stationary handle portion 20. A drive plate 32 (FIGS. 4 and 5) includes a slotted opening 34 dimensioned to receive a feed bar 36 which is slidably positioned within body 26 and elongated body portion 14. A pair of feed bar guide members 38 (FIGS. 6-8) are supported at spaced locations between half-sections 26a and 26b. Each guide member 38 includes a slot 38a for slidably receiving feed bar 36.

A biasing member 40, which is preferably a torsion spring, is positioned about feed bar 36 between the forward most guide member 38 and drive plate 32. Biasing member 40 is positioned to urge drive plate 32 proximally into engagement with trigger 22 to urge trigger 22 in a counter-clockwise direction as shown in FIG. 3 to a (non-compressed) position spaced from stationary handle portion 20. Trigger 22 includes a flat face 44 against which drive plate 32 abuts.

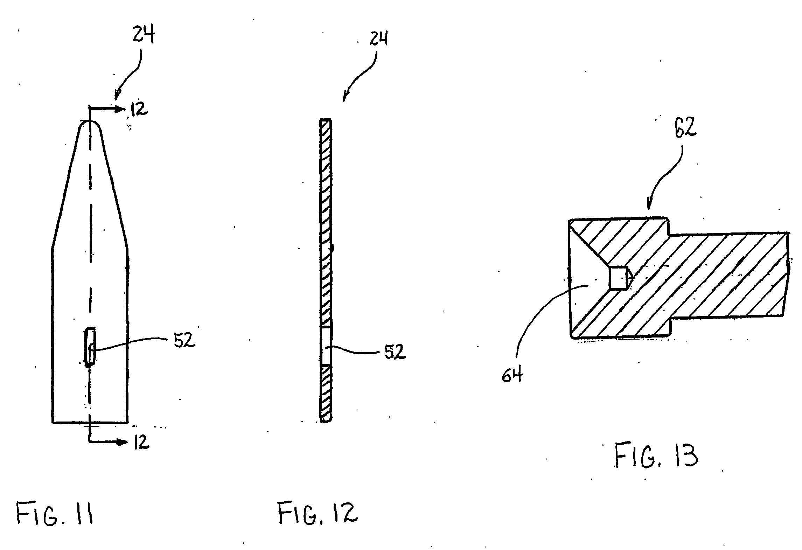

Referring also to FIGS. 9 and 10, feed bar 36 preferably includes a flat elongated member having a substantially rectangular cross-section with radiused top and bottom edges. Alternately, other feed bar configurations are envisioned, e.g., cylindrical, triangular, square, etc. Feed bar 36 extends through the proximal and distal ends of barrel portion 18 of handle assembly 12. The distal end of feed bar 36 extends into elongated body portion 14. As discussed above, feed bar 36 is slidably supported within barrel portion 18 of handle assembly 12 by guide members 38. See FIG. 3. The proximal end of feed bar 36 extends rearwardly from barrel portion 18 and includes a knob or grip 50 secured to the proximal end thereof. Knob 50 is configured to be grasped by a surgeon such that feed bar 36 can be returned to a retracted position. Knob 50 also functions as a stop to limit distal advancement of feed bar 36.

Locking member 24 is pivotally supported within the distal end of barrel portion 18 of handle assembly 12 and includes an elongated slot 52 (FIGS. 11 and 12) dimensioned to slidably receive feed bar 36. A biasing member 56, which is preferably a torsion spring, is positioned to urge locking member 24 from a substantially vertical orientation in which slot 52 is aligned with feed bar 36 to allow feed bar 36 to slide in relation thereto to a canted orientation in which the top and/or bottom edges defining slot 52 engage feed bar 36 to prevent proximal movement of feed bar 36 in relation to locking member 24. Because of the angle of locking member 24 in relation to feed bar 36 in its canted position, locking member 24 permits distal advancement of feed bar 36 when trigger 22 is actuated. The top surface of feed bar 36 may be roughened, e.g., serrated, scarred, knurled, etc., to enhance contact between locking member 24 and feed bar 36.

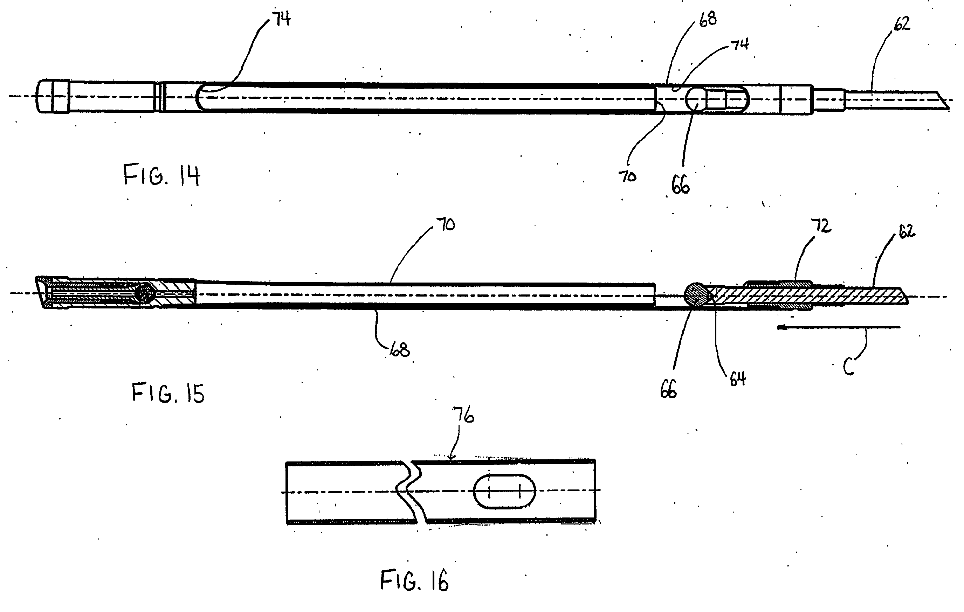

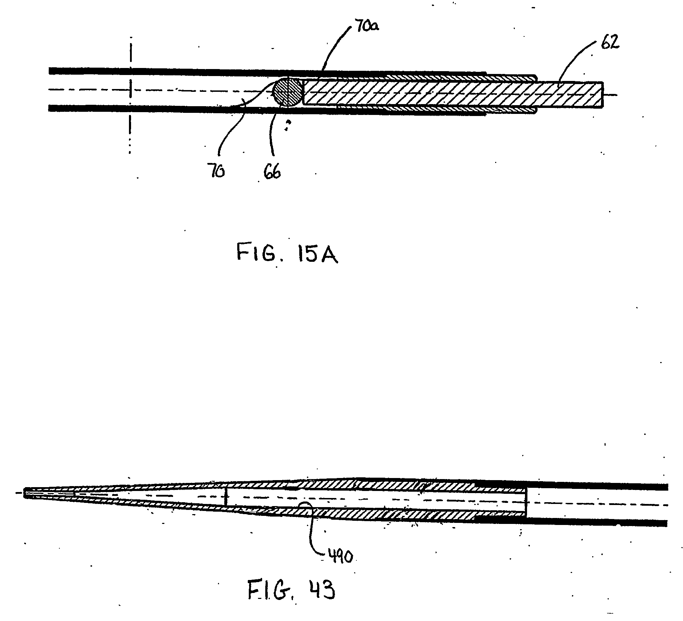

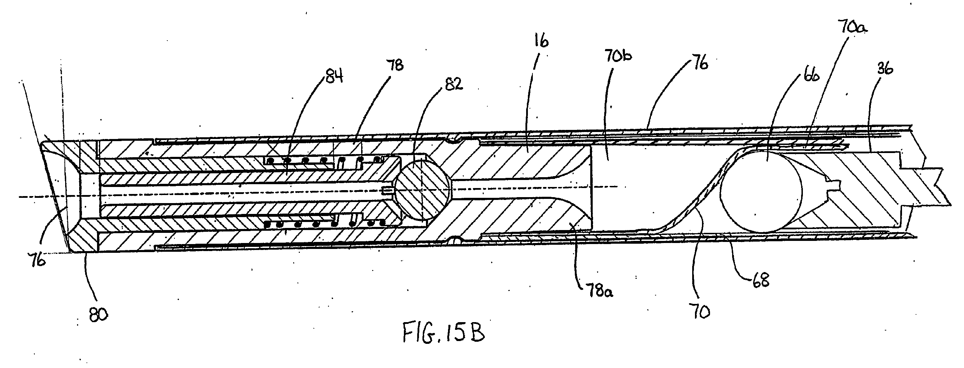

Referring to FIGS. 13-15, the distal end of feed bar 36 is secured to the proximal end of a pusher 62 in a known manner, e.g., welding, brazing, screws, etc. Alternately, feed bar 36 and pusher 62 may be monolithically formed. The distal end of pusher 62 includes a cavity 64 configured and dimensioned to receive a drive member 66 (FIGS. 14 and 15). Drive member 66 preferably has a spherical configuration and is made of non-stick material such as a synthetic fluorine containing resin, e.g., Teflon^{™} or formed of another material, e.g., plastic, metal, etc., that has a non-stick outer surface, layer or covering. Alternately, drive members having different configurations are envisioned, e.g., cylindrical, rectangular, convex, concave, etc. Preferably, drive member 66 is not physically attached to pusher 62. Alternately, drive member 66 can be monolithically formed with pusher 62 or fastened to pusher 36 within cavity 64 using any known technique including welding, brazing, adhesives, etc.

Referring to FIGS. 1-3 and 14-16, elongated body 14 includes an inner tube 68, a wound closure material reservoir 70, a pusher guide 72 and an outer tube 76. Reservoir 70 is preferably a tube having two open ends. Reservoir 70 can, for example be a capillary tube or cartridge. Alternately, reservoir 70 may be formed from a flexible, deformable or compressible material having an open distal end 70b and a sealed proximal end 70a. The sealed proximal end 70a may be pinch sealed between inner and outer tubes 68 and 76, respectively, to permit drive member 66 to pass over the proximal end of the outer surface of reservoir 70 to force wound closure material from distal end 70b of reservoir 70. See FIG. 15A. Open distal end 70b of reservoir 70 may be positioned about the proximal end 78a of housing 78 of tip assembly 16 within inner tube 68. See FIG. 15B. Such a reservoir can be deformed by direct contact of drive member 66 with reservoir 70 to dispense material. Preferably, distal end 70b of reservoir 70 is fastened about the proximal end 78a of tip assembly 16 such as by crimping, press-fitting, adhesives, etc. Alternately, other fastening techniques may be used.

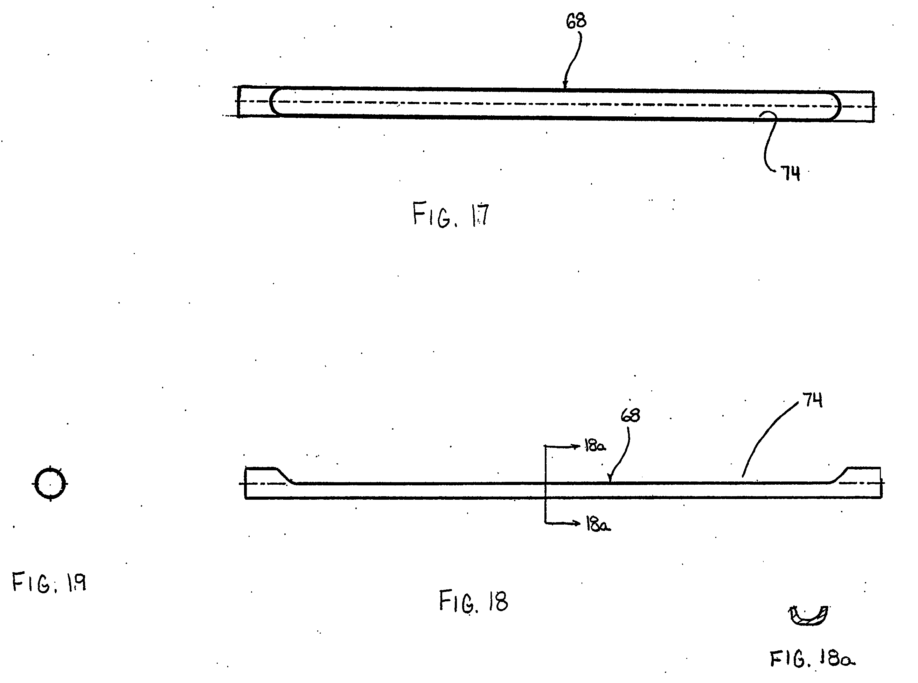

Inner tube 68 includes an elongated opening 74 formed therein. (See FIGS. 17-19). Opening 74 is dimensioned to receive or allow passage of reservoir 70 during assembly of applicator 10. The distal end of inner tube 68 is fastened about proximal end 78a of tip assembly 16 and the distal end of reservoir 70. The proximal end of inner tube 68 is fastened about pusher guide 72. Referring to FIGS. 1-3 and 16, an outer tube 76 extends between handle assembly 12 and tip assembly 16 to axially fix handle assembly 12 and tip assembly 16 in relation to each other and confine inner tube 68 and reservoir 70 therein.

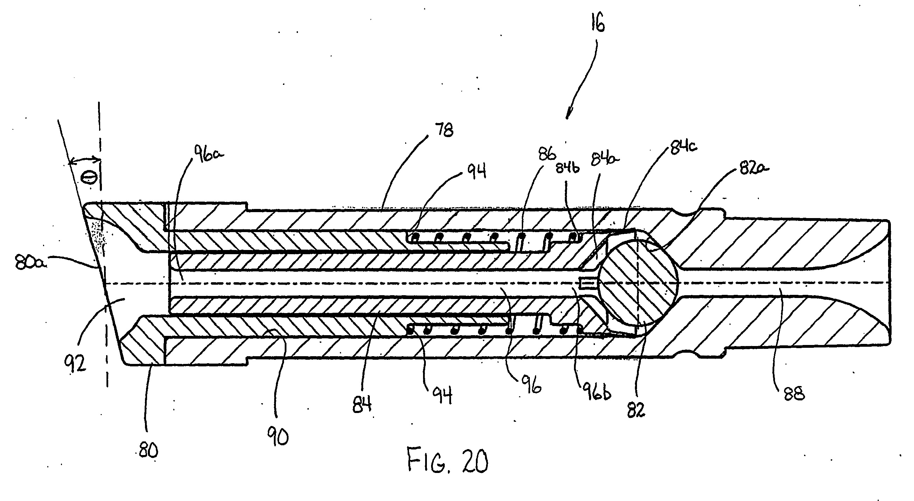

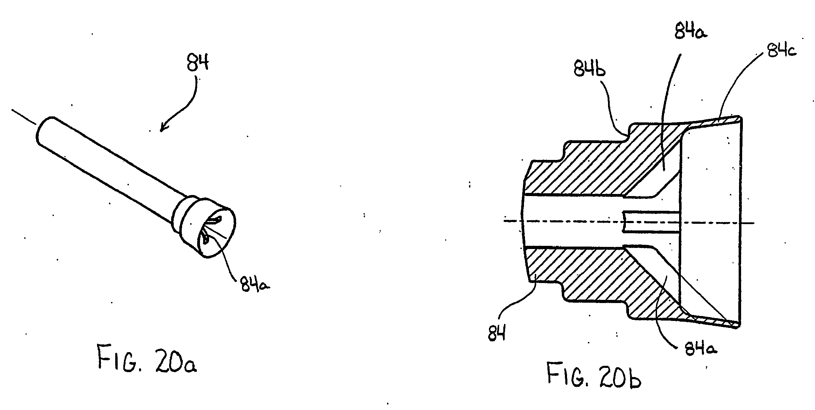

Referring to FIGS. 20-20b, tip assembly 16 includes housing 78, a tip member 80, a valve member 82, a seal member 84 and a biasing member 86. Housing 78 includes a proximal end defining an inlet channel 88 which converges towards valve member 82 and a distal end defining a hollow bore 90. Alternately, channel 88 may include other configurations, e.g., cylindrical, diverging, etc. An internal wall of housing 78 adjacent inlet channel 88 defines a valve seat 82a. Tip member 80 is fixedly received within bore 90 of housing 78 using press-fitting, adhesives, welding, brazing, etc. The distal end of tip member 80 includes a spherical or concave receptacle 92 which will be discussed in detail below and an angled distal face 80a. Angled face 80a defines an angle θ with respect to an axis perpendicular to the longitudinal axis of the applicator of between about 10° and about 45° and preferably about 15°. The proximal end of tip member 80 has a reduced outer diameter defining a step or shoulder 94. Seal member 84 is slidably positioned within tip member 80 and includes an elongated central discharge channel 96 having a distal end 96a located adjacent receptacle 92 of tip member 80 and a , proximal end 96b defining a recess having a plurality of inlet ports 84a. Seal member 84 and valve member 82 can be integrally formed or formed separately. The wall defining central discharge channel 96 is preferably coated with a non-stick material such as a synthetic fluorine containing resin, e.g., Teflon^{™}. Valve member 82 may also include a non-stick coating. Seal member 84 also includes a shoulder 84b and a resilient annular, outwardly diverging finger 84c. Biasing member 86, which is preferably a torsion spring, is positioned between shoulder 94 of tip member 80 and shoulder 84b of seal 84 to urge seal member 84 including valve member 82 into sealing contact with valve seat 82a of housing 78.

Referring to FIGS. 3, 15 and 20, when trigger 22 is pivoted about pivot member 28 in the direction indicated by arrow "A" in FIG. 3, drive plate 32 is tilted into engagement with feed bar 36 and moved forwardly in the direction indicated by arrow "B" in FIG. 3 to effect distal advancement of feed bar 36. As feed bar 36 is advanced distally in the direction indicated by arrow "C" in FIG. 15, drive member 66 is also advanced distally into and through reservoir 70 to force wound closure material to flow into inlet channel 88 of tip assembly seal member 84. As trigger 22 is pivoted further, the fluid pressure of the wound closure material within channel 88 increases until the force applied by spring 86 on valve member 82 is overcome and valve member 82 is lifted off of valve seat 82a. When this occurs, valve member 82 advances seal member 84 distally against the bias of spring member 86 such that the distal end of seal member 84 is positioned adjacent to or extends into receptacle 92 of tip assembly 16. Thereafter, wound closure material flows around valve member 82 through channels 84a in seal member 84 into central channel 96 of seal member 84 into receptacle 92 of tip assembly 16 and can be dispensed on tissue. Referring again to FIG. 3, when a surgeon releases trigger 22, biasing member 40 urges trigger 22 to pivot in the direction indicated by arrow "D" to its non-compressed position to disengage drive plate 32 from feed bar 36. Locking member 24 remains in engagement with feed bar 36 to prevent retraction of feed bar 36. In order to retract feed bar 36, locking member 24 is moved against the bias of spring 56 to its vertical position disengaged from feed bar 36 and knob 50 is pulled proximally. Trigger 22 can be actuated multiple times to dispense the required amount of wound closure material.

After a sufficient amount of wound closure material has been dispensed and trigger 22 is released by a surgeon, spring 86 will return valve member 82 onto valve seat 82a. Movement of valve member 82 proximally back onto valve seat 82a draws a vacuum within central discharge channel 96 of seal member 84 to draw some of the wound closure material within receptacle 92 of tip 80 back into central discharge channel 96 to minimize the amount of drippage from the instrument.

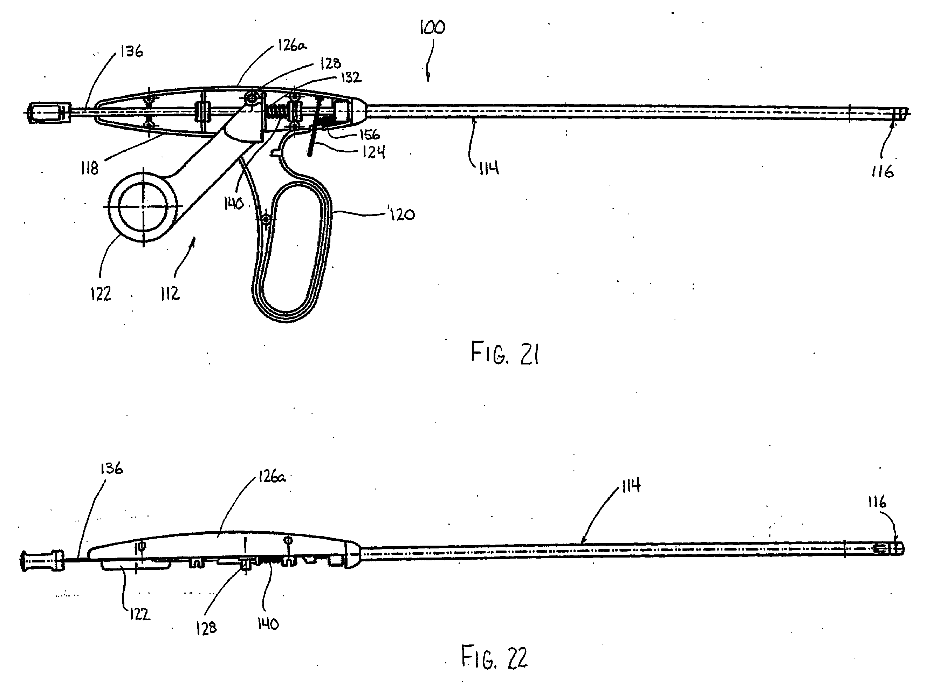

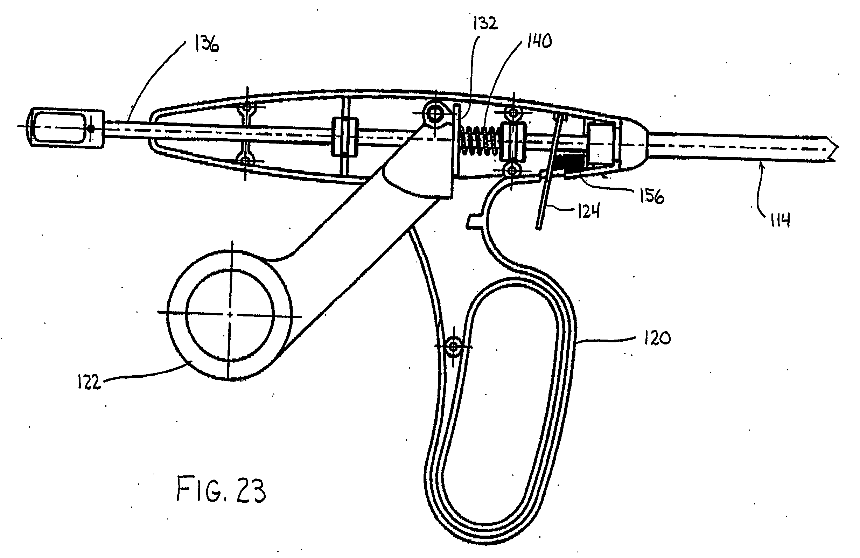

FIGS. 21-23 illustrate another preferred embodiment of the presently disclosed wound closure material applicator shown generally as 100. Wound closure material applicator 100 includes a handle assembly 112, an elongated body portion 114, and a tip assembly 116 supported on the distal end of elongated body portion 114. Handle assembly 112 is similar to handle assembly 12 and will only be discussed in detail to the extent necessary to identify differences in construction and operation.

Handle assembly 112 includes a stationary handle portion 120, a movable handle portion or trigger 122, and a locking member 124. Trigger 122 is pivotally supported between body half-sections 126a (only one half-section is shown) about a pivot member 128 and is in operative engagement with a drive plate 132. A biasing member 140 is positioned to urge drive plate 132 and trigger 122 to a rearward or non-compressed position. Trigger 122, drive plate 132 and biasing member 140 are associated with feed bar 136 and operate in a manner substantially similar to trigger 22, drive plate 32 and biasing member 40 of applicator 10 described above and will not be discussed in further detail herein.

Handle assembly 112 includes a locking member 124 which is pivotally supported in the distal end of barrel portion 118 of handle assembly 112 and a biasing member 156 which urges locking member 124 into a canted position engaged with feed bar 136. Although the location of locking member 124 and biasing member 156 has changed slightly compared to that of locking member 24 and biasing member 56 described above, the operation and function of locking member 24 is identical to that described above and will not be described in further detail herein.

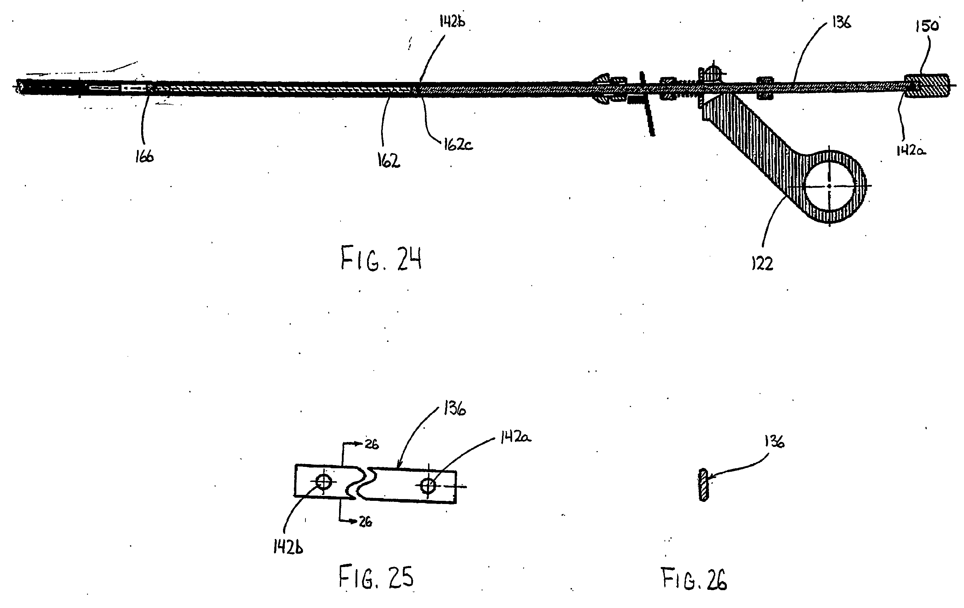

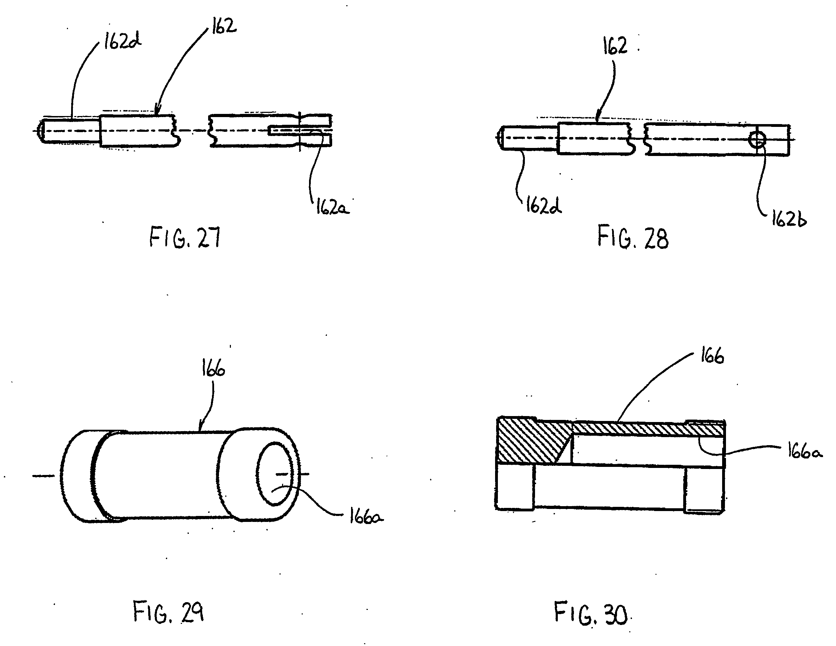

Referring to FIGS. 24-26, feed bar 136 preferably includes a flat elongated member having a substantially rectangular cross-section with radiused top and bottom edges. Alternately as discussed above, other configurations are envisioned. The proximal and distal ends of feed bar 136 preferably include throughbores 142a and 142b, respectively, dimensioned to receive screws or pins. The proximal end of feed bar 136 is secured to knob 150 and the distal end of feed bar 136 is secured to pusher 162.

Referring also to FIGS. 27 and 28, pusher 162 is preferably substantially cylindrical and includes a proximal end having a transverse slot 162a configured and dimensioned to receive the distal end of feed bar 136. A throughbore 162b formed in the proximal end of pusher 162 is dimensioned to receive a screw or pin 162c for securing feed bar 136 to pusher 162. Alternately other fastening techniques, such as welding, brazing, crimping, etc., may be sued to secure the feed bar 136 to the pusher.

Referring to FIGS. 24 and 29-31, a piston 166 is secured to the distal end of pusher 162. Piston 166 has a bore 166a formed in a proximal end thereof. Bore 166a is dimensioned to receive a reduced diameter portion 162d of pusher 162. Pusher 162 is secured within bore 166a of piston 166 using any known fastening technique including press-fitting, screws, pins, welding, etc. Alternately, piston 166 and pusher 162 can be monolithically formed. The distal end of piston 166 is cylindrical and corresponds in shape to the internal wall of wound closure material reservoir 170.

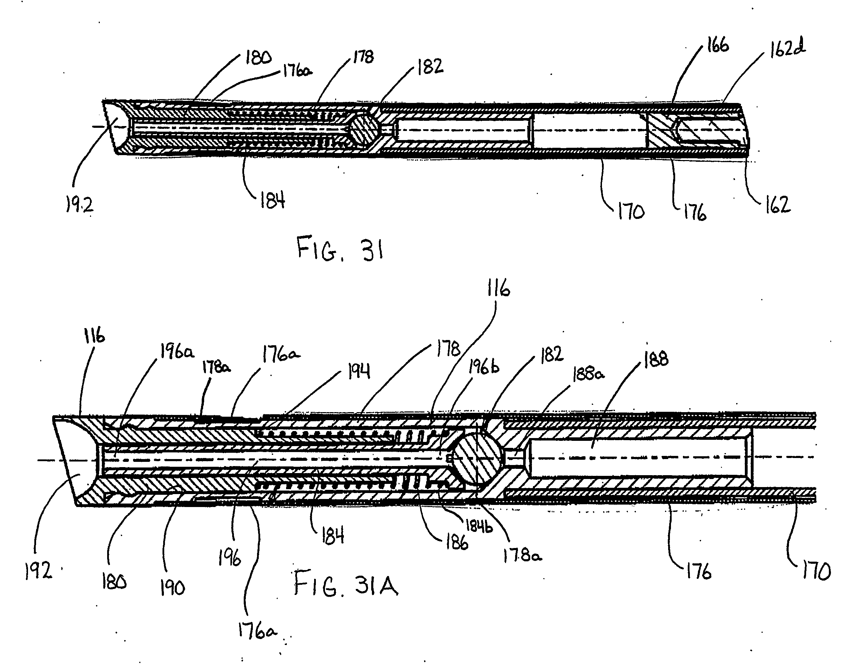

Referring to FIGS. 31 and 31A, elongated body 114 includes an inner tube 170 defining a body channel and an outer tube 176. Inner tube 170 includes a supply of wound closure material (not shown) which may be in the form of a cartridge of material or material located directly in the channel. The distal end of inner tube 170 is fixedly positioned about the proximal end of housing 178 of tip assembly 116. Outer tube 176 extends between handle assembly 112 and tip assembly 116 and encloses elongated body 114 and tip assembly 116. The distal end of outer tube 176 includes spring tabs 176a which are received in recesses 178a formed in housing 178 of tip assembly 116 and axially secure tip assembly 116 within outer tube 116a.

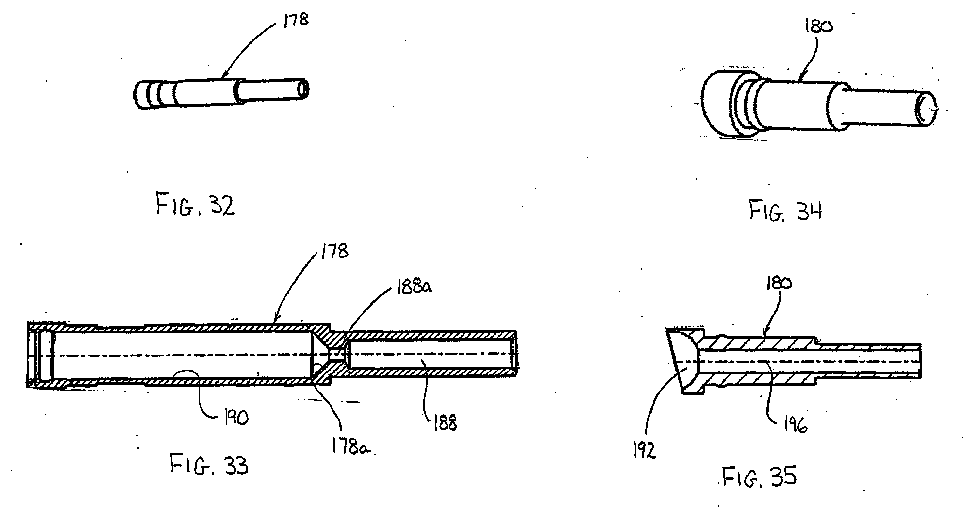

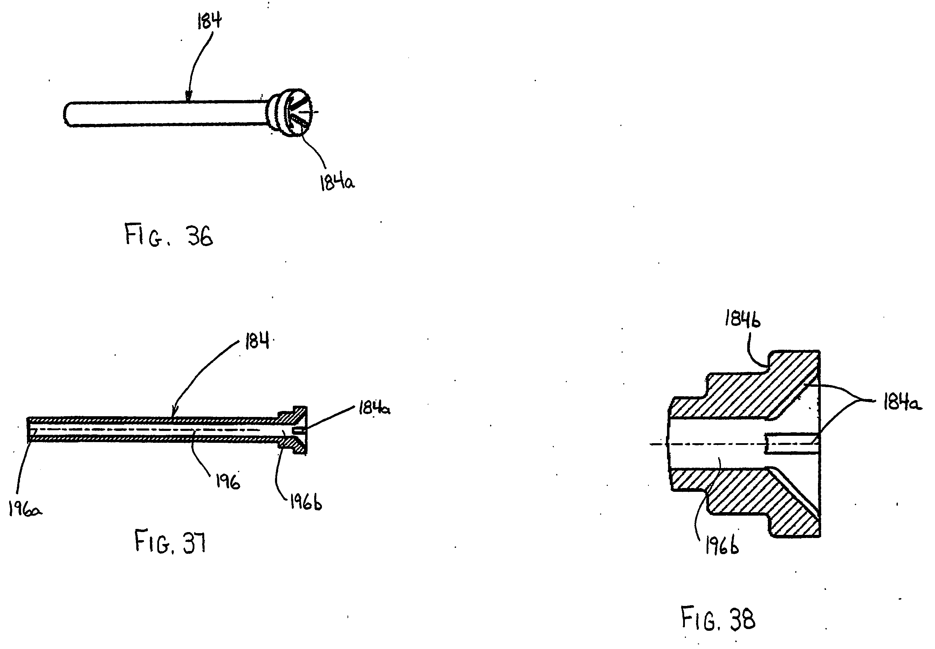

Tip assembly 116 includes a housing 178, a tip member 180, a valve member 182, a seal member 184 and a biasing member 186. Housing 178 includes a proximal end defining an inlet channel 188 which includes a convergent, reduced diameter section 188a which extends towards valve member 182 and a distal end defining a hollow bore 190. See FIGS. 32 and 33. A central portion of housing 178 defines a valve seat 178a. Tip member 180 is fixedly received within bore 190 of housing 178. The distal end of tip member 180 includes a spherical receptacle 192 which will be discussed in detail below. See FIGS. 34 and 35. The proximal end of tip member 180 has a reduced outer diameter defining a step or shoulder 194. Seal member 184 is slidably positioned within tip member 180 and includes an elongated central discharge channel 196 having a distal end 196a located adjacent receptacle 192 of tip member 180 and a proximal end 196b defining a recess having a plurality of inlet ports 184a (FIG. 38) which communicate with discharge channel 196. The wall defining central channel 196 is preferably coated with a non-stick synthetic fluorine containing, e.g., Teflon^{™} material. Seal member 184 also includes a shoulder 184b. Biasing member 186, which is preferably a torsion spring, is positioned between shoulder 194 of housing 178 and shoulder 184b of seal member 184 to urge seal member 184 into sealing contact with valve seat 178a of housing 178.

In use, when trigger 122 is pivoted about pivot member 128 (FIG. 21), drive plate 132 is tilted into engagement with feed bar 136 and moved forwardly to effect distal advancement of feed bar 136. As feed bar 136 is advanced distally, pusher 162 and drive member 166 are advanced distally through inner tube or reservoir 170 to force wound closure material to flow into inlet channel 188 of housing 178. As trigger 22 is pivoted further, the fluid pressure of the wound closure material within channel 188 increases until the force applied by spring 186 on valve member 182 is overcome and valve member 182 lifts off of valve seat 178a. When this occurs, wound closure material flows around valve member 182 through ports 184a in seal member 184 into central discharge channel 196 of seal member 184 and into receptacle 192 of tip assembly 116. Thereafter, wound closure material can be dispensed on tissue. As discussed above with respect to wound closure material applicator 10, when trigger 122 is released and the pressure of wound closure material in channel 188 is reduced, valve member 182 is urged into contact with valve seat 178a to seal inlet channel 188. As valve member 182 is moved towards valve seat 178a, a vacuum is created in center discharge channel 196 of seal member 184 which draws wound closure material positioned in spherical receptacle 192 into central channel 196 to minimize dripping of wound closure material from tip assembly 116.

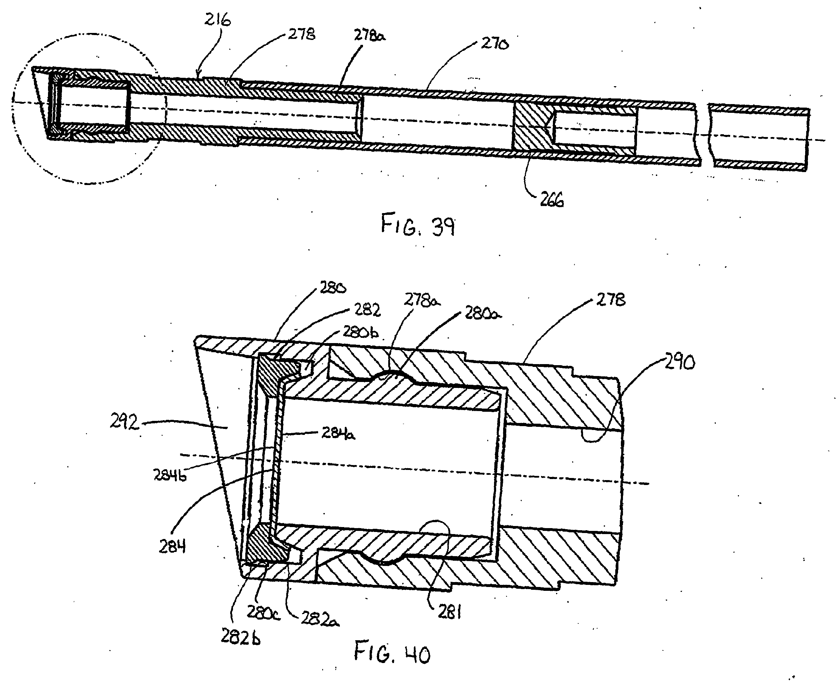

FIGS. 39 and 40 illustrate another preferred embodiment of the tip assembly shown generally as 216. Tip assembly 216 includes a housing 278, a tip member 280, an insert member 282 and a permeable membrane 284. The proximal end of housing 278 is fixedly received within the distal end of a wound closure material reservoir 270 in a manner similar to that disclosed above. Tip member 280 is fixedly received within the distal end of housing 278. Preferably, protrusions 280a are formed on an outer surface of tip member 280 and are received within concavities 278a formed on an inner wall of housing 278 to secure tip member 280 to tip housing 278. Alternately, other known securement means may be used to secure tip member 280 to housing 278, e.g., press-fitting, crimping, adhesives, etc.

Insert member 282 includes an annular ring having a proximally extending tapered portion 282a and a distally located increased diameter portion 282b. Tip member 280 includes an annular recess 280b configured to receive tapered portion 282a of insert member 282 and a radial cutout 280c configured to receive increased diameter portion 282b of insert member 282. Insert member 282 is received within tip member 280 to secure permeable membrane 284 between a front face of tip member 280 and a proximal surface, e.g., 282a, of insert member 282.

Tip member 280 may be substituted for the tip assemblies discussed above with reference to wound closure material applicators 10 and 100. In use, when piston 266 is advanced distally through reservoir 270, wound closure material is forced from reservoir 270 into bore 290 of tip housing 278 and bore 281 of tip member 280 into contact with on interior side 284a of permeable membrane 284. As piston 266 is advanced and the pressure of the wound closure material within bore 281 increases, the wound closure material will pass from interior side 284a through permeable membrane 284 to exterior side 284b into receptacle 292 at the distal end of tip member 280. Thereafter, the wound closure material can be dispensed on tissue by the surgeon.

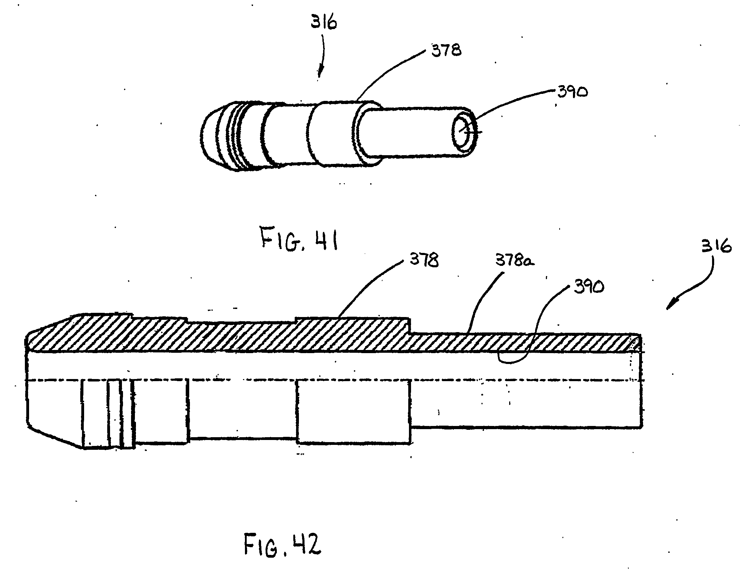

FIGS. 41 and 42 illustrate yet another preferred embodiment of the tip assembly shown generally as 316. Tip assembly 316 includes a tip housing 378 having a central throughbore 390. The proximal end of tip housing 378 includes a reduced diameter portion 378a which is configured and dimensioned to be fixedly received within the distal end of a wound closure material reservoir (such as reservoir 170, FIG. 31A). The outer surface of tip housing 378 also includes one or more recesses for engaging the outer housing of a wound closure material applicator. Tip assembly 316 is suitable for use with wound closure material applicators having a more viscous wound closure material than are tip assemblies 16, 116 and 216 disclosed above.

It is understood that would closure materials include but are not limited to adhesives, hemostats and sealants. Adhesives function to attach or hold organs, tissues or structures , sealants to prevent fluid leakage, and hemostats to halt or prevent bleeding. Examples of adhesives which can be employed include protein derived, aldehyde-based adhesive materials, for example, the commercially available albumin/glutaraldehyde materials sold under the trade designation BioGlue^{™} by Cryolife, Inc., and cyanoacrylate-based materials sold under the trade designations Indermil^{™} and Derma Bond^{™} by Tyco Healthcare Group, LP and Ethicon Endosurgery, Inc., respectively. Examples of sealants which can be employed include fibrin sealants and collagen-based and synthetic polymer-based tissue sealants. Examples of commercially available sealants are synthetic polyethylene glycolbased, hydrogel materials sold under the trade designation CoSeal^{™} by Cohesion Technologies and Baxter International, Inc. Examples of hemostat materials which can be employed include fibrin-based, collagen-based, oxidized regenerated cellulose-based and gelatin-based topical hemostats herein can include astringents and coagulants. Examples of commercially available hemostat materials are fibrinogen-thrombin combination materials under sold the trade designations CoStasis^{™} by Tyco Healthcare Group, LP and Tisseel^{™} sold by Baxter International, Inc. Hemostats herein include astringents, e.g., sulphates of aluminum, and coagulants.

It will be understood that various modifications may be made to the embodiments disclosed herein. For example, the tip housing may define a converging throughbore 490. See FIG. 43. The applicator may be constructed from any material or materials suitable for surgical use including metals, stainless steel, plastics, polymeric materials, PTFE, etc. Any part or all of the applicator may be formed so as to be disposable. For example, the elongated body and/or tip assembly may be in the form of a replaceable and disposable unit. Although, especially suitable for dispensing wound closure material to apply mesh to tissue during a surgery for hernia repair, the wound closure material applicator may be used in a variety of surgical procedures requiring wound closure materials. Further, the above-described applicator may also be suitable for dispensing materials other than wound closure materials in surgical and non-surgical applications. Therefore, the above description should not be construed as limiting, but merely as exemplifications of preferred embodiments.

## Claims

1. A wound closure material applicator for applying a wound closure material comprising:
a handle assembly (12) including a moveable handle (22);
a body portion (14) extending distally from the handle assembly, the body portion defining a body channel;
a tip assembly (16) supported adjacent the distal end of the body portion, the tip assembly including a seal member (84), a valve seat (82a) and a discharge channel (96) positioned distally of the valve seat, the seal member being moveable distally of a closed position in which the seal member engages the valve seat to an open position in which the seal member is spaced from the valve seat, wherein movement of the seal member from the open position to the closed position causes a reduction of pressure within the discharge channel to minimize the drippage of wound closure material from the tip assembly
**characterized in that**
the seal member is an assembly that comprises a sealing member (84) located radially around the discharge channel and a valve member (82) positioned at a proximal end of the sealing member such that the sealing member presses on the valve member and in the closed position, the valve member engages the valve seat and
further **characterized in that**
the seal member includes at least one inlet port (84a) positioned distally of the valve member, the at least one inlet port communicating with the discharge channel.

2. A wound closure material applicator according to claim 1, wherein the valve member (82) is a substantially spherical valve member.

3. A wound closure material applicator according to either of the preceding claims, wherein the tip assembly includes a housing (78) defining a bore (90), an inlet channel (88) and the valve seat, the seal member being positioned within the bore.

4. A wound closure material applicator according to claim 3, wherein the inlet channel converges from its proximal to its distal end.

5. A wound closure material applicator according to any one of the preceding claims, wherein the tip assembly further includes a tip member (80) having a concave receptacle (92) in communication with the discharge channel.

6. A wound closure material applicator according to claim 5, wherein the concave receptacle is substantially spherical.

7. A wound closure material applicator according to any one of the preceding claims wherein the seal member includes a annular resilient finger (84c) which is positioned to engage a wall defining the bore of the housing, the annular finger providing a seal between the seal member and the wall defining bore.

8. A wound closure material applicator according to any one of the preceding claims, wherein the at least one inlet port is a plurality of inlet ports.

9. A wound closure material applicator according to any one of the preceding claims, further including a feed bar (36) moveably supported within the body portion, the feed bar being operatively associated with the moveable handle (22) such that actuation of the moveable handle effects distal movement of the feed bar within the body portion.

10. A wound closure material applicator according to claim 9, further including a pusher (62) supported on the distal end of the feed bar within the body portion, the moveable handle being actuable to move the feed bar and pusher distally to force a wound closure material from the body portion into the tip assembly.

11. A wound closure material applicator according to claim 10, wherein the pusher is formed integrally with the feed bar.

12. A wound closure material applicator according to claim 9, 10 or 11, wherein the feed bar extends through the handle assembly and a drive plate (32) positioned within the handle assembly, the moveable handle being actuable to move the drive plate into engagement with the feed bar to move the feed bar distally.

13. A wound closure material applicator according to claim 12, further including a locking plate (24) positioned within the handle assembly and having a slot (52) formed therein, the feed bar extending through the slot, the locking plate being moveable from a first position permitting the feed bar to move through the slot to a second position engaging the feed bar to prevent proximal movement of the feed bar in relation to the locking plate.

14. A wound closure material applicator according to claim 13, further including a biasing member (56) positioned to urge the locking plate to the second position.

15. A wound closure material applicator according to any one of the preceding claims, including the wound closure material.

16. A wound closure material applicator according to claim 15, wherein the wound closure material is cyanoacrylate adhesive.

17. A wound closure material applicator according to any one of the preceding claims, wherein the tip assembly includes a biasing member (86) that urges the seal member to the closed position.

18. A wound closure material applicator according to any one of the preceding claims, wherein the body channel defines a reservoir for wound closure material.

## Patentansprüche

1. Wundverschlussmaterialapplikator zum Applizieren eines Wundverschlussmaterials umfassend:
eine Griffanordnung (12)umfassend einen bewegbaren Griff (22);
einen Körperabschnitt (14), welcher sich distal von der Griffanordnung aus erstreckt, wobei der Körperabschnitt einen Körperkanal definiert;
eine Spitzenanordnung (16), welche in der Nähe des distalen Endes des Körperabschnitts gehalten ist, wobei die Spitzenanordnung ein Abdichtungselement (84), einen Ventilsitz (82a) und einen distal des Ventilsitzes positionierten Auslasskanal (96) umfasst, wobei das Dichtungselement aus einer geschlossenen Position, in der das Dichtungselement mit dem Ventilsitz eingreift, distal in eine geöffnete Position, in der das Dichtungselement von dem Ventilsitz beabstandet ist, bewegbar ist, wobei die Bewegung des Dichtungselements von der geöffneten Position in die geschlossene Position eine Reduktion des Druckes innerhalb des Auslasskanals hervorruft, um das Heraustropfen des Wundverschlussmaterials aus der Spitzenanordnung zu minimieren,
**dadurch gekennzeichnet,**
**dass** das Dichtungselement eine Anordnung ist, die ein radial um den Auslasskanal herum angeordnetes Dichtungselement (84) und ein Ventilelement (82), welches an einem proximalen Ende des Dichtungselements so positioniert ist, dass das Dichtungselement auf das Ventilelement presst und das Ventilelement in der geschlossenen Position mit dem Ventilsitz eingreift, umfasst, und
weiter **dadurch gekennzeichnet,**
**dass** das Dichtungselement zumindest einen distal des Ventilelements positionierten Einlassanschluss (84a) umfasst, wobei der zumindest eine Einlassanschluss mit dem Auslasskanal kommuniziert.

2. Wundverschlussmaterialapplikator gemäß Anspruch 1, wobei das Ventilelement (82) ein im Wesentlichen kugelförmiges Ventilelement ist.

3. Wundverschlussmaterialapplikator gemäß einem der vorstehenden Ansprüche, wobei die Spitzenanordnung ein eine Bohrung (90) definierendes Gehäuse (78), einen Einlasskanal (88) und den Ventilsitz umfasst, wobei das Dichtungselement innerhalb der Bohrung positioniert ist.

4. Wundverschlussmaterialapplikator gemäß Anspruch 3, wobei der Einlasskanal von seinem proximalen Ende aus zu seinem distalen Ende hin konvergiert.

5. Wundverschlussmaterialapplikator gemäß einem der vorstehenden Ansprüche, wobei die Spitzenanordnung weiterhin ein Spitzenelement (80) umfasst, welches eine konkave Aufnahme (92) in Kommunikation mit dem Auslasskanal aufweist.

6. Wundverschlussmaterialapplikator gemäß Anspruch 5, wobei die konkave Aufnahme im Wesentlichen kugelförmig ist.

7. Wundverschlussmaterialapplikator gemäß einem der vorstehenden Ansprüche wobei das Dichtungselement einen ringförmigen, elastischen Finger (84c) umfasst, der so positioniert ist, dass er mit einer die Bohrung des Gehäuses definierenden Wand eingreift, wobei der ringförmige Finger eine Dichtung zwischen dem Dichtungselement und der die Bohrung definierenden Wand ausbildet.

8. Wundverschlussmaterialapplikator gemäß einem der vorstehenden Ansprüche, wobei der zumindest eine Einlassanschluss eine Mehrzahl von Einlassanschlüssen ist.

9. Wundverschlussmaterialapplikator gemäß einem der vorstehenden Ansprüche, weiterhin umfassend eine Zuführstange (36), welche bewegbar innerhalb des Körperabschnitts gehalten ist, wobei die Zuführstange so mit dem bewegbaren Griff (22) zusammenwirkend verbunden ist, dass eine Betätigung des bewegbaren Griffes eine distale Bewegung der Zuführstange innerhalb des Körperabschnitts hervorruft.

10. Wundverschlussmaterialapplikator gemäß Anspruch 9^, weiterhin umfassend einen Drücker (62), welcher an dem distalen Ende der Zuführstange innerhalb des Körperabschnitts gehalten ist, wobei der bewegbare Griff so betätigbar ist, dass er die Zuführstange und den Drücker distal bewegt, um ein Wundverschlussmaterial aus dem Körperbereich heraus in die Spitzenanordnung zu zwingen.

11. Wundverschlussmaterialapplikator gemäß Anspruch 10, wobei der Drücker integral mit der Zuführstange geformt ist.

12. Wundverschlussmaterialapplikator gemäß Anspruch 9, 10 oder 11, wobei sich die Zuführstange durch die Griffanordnung und eine innerhalb der Griffanordnung positionierte Antriebsplatte (32) hindurch erstreckt, wobei der bewegbare Griff betätigbar ist, um die Antriebsplatte in Eingriff mit der Zuführstange zu bewegen, um die Zuführstange distal zu bewegen.

13. Wundverschlussmaterialapplikator gemäß Anspruch 12, weiterhin umfassend eine Verschlussplatte (24), welche innerhalb der Griffanordnung positioniert ist und welche einen darin eingeformten Schlitz (52) aufweist, wobei sich die Zuführstange durch den Schlitz hindurch erstreckt, wobei die Verschlussplatte von einer ersten Position, die es der Zuführstange erlaubt, sich durch den Schlitz hindurch zu bewegen, in eine zweite Position, in der sie mit der Zuführstange eingreift, bewegbar ist, um eine proximale Bewegung der Zuführstange bezüglich der Verschlussplatte zu verhindern.

14. Wundverschlussmaterialapplikator gemäß Anspruch 13, weiterhin umfassend ein Vorspannelement (56), das so positioniert ist, dass es die Verschlussplatte in die zweite Position zwingt.

15. Wundverschlussmaterialapplikator gemäß einem der vorstehenden Ansprüche umfassend das Wundverschlussmaterial.

16. Wundverschlussmaterialapplikator gemäß Anspruch 15, wobei das Wundverschlussmaterial ein Cyanoacrylat-Klebstoff ist.

17. Wundverschlussmäterialapplikator gemäß einem der vorstehenden Ansprüche, wobei die Spitzenanordnung ein Vorspannelement (86) umfasst, welches das Dichtungselement in die geschlossene Position zwingt.

18. Wundverschlussmaterialapplikator gemäß einem der vorstehenden Ansprüche, wobei der Körperkanal ein Reservoir für das Wundverschlussmaterial definiert.

## Revendications

1. Applicateur de matière de fermeture de plaie pour appliquer une matière de fermeture de plaie comprenant :
un ensemble de poignées (12) comprenant une poignée mobile (22);
une portion de corps (14) s'étendant distalement de l'ensemble de poignées, la portion de corps définissant un canal de corps ;
un ensemble de pointe (16) supporté d'une manière adjacente à l'extrémité distale de la portion de corps, l'ensemble de pointe comprenant un élément de scellement (84), un siège de soupape (82a) et un canal d'évacuation (96) positionné distalement du siège de soupape, l'élément de scellement étant déplaçable distalement d'une position fermée dans laquelle l'élément de scellement vient en prise avec le siège de soupape à une position ouverte dans laquelle l'élément de scellement est espacé du siège de soupape, où le mouvement de l'élément de scellement de la position ouverte à la position fermée entraîne une réduction de pression dans le canal d'évacuation pour réduire à un minimum l'égouttement de la matière de fermeture de plaie de l'ensemble de pointe,
**caractérisé en ce que**
l'élément de scellement est un ensemble qui comprend un élément de scellement (84) situé radialement autour du canal d'évacuation et un élément de soupape (82) positionné à une extrémité proximale de l'élément de scellement de telle sorte que l'élément de scellement exerce une pression sur l'élément de soupape et, dans la position fermée, l'élément de soupape vient en prise avec le siège de soupape et
**caractérisé en outre en ce que**
l'élément de scellement comprend au moins un orifice d'admission (84a) positionné distalement de l'élément de soupape, au moins un orifice d'admission précité communiquant avec le canal d'évacuation.

2. Applicateur de matière de fermeture de plaie selon la revendication 1, où l'élément de soupape (82) est un élément de soupape sensiblement sphérique.

3. Applicateur de matière de fermeture de plaie selon l'une quelconque des revendications précédentes, où l'ensemble de pointe comprend un boîtier (78) définissant un perçage (90), un canal d'admission (88) et le siège de soupape, l'élément de scellement étant positionné dans le perçage.

4. Applicateur de matière de fermeture de plaie selon la revendication 3, où le canal d'admission converge de son extrémité proximale à son extrémité distale.

5. Applicateur de matière de fermeture de plaie selon l'une quelconque des revendications précédentes, où l'ensemble de pointe comprend en outre un élément de pointe (80) ayant un réceptacle concave (92) en communication avec le canal d'évacuation.

6. Applicateur de matière de fermeture de plaie selon la revendication 5, où le réceptacle concave est sensiblement sphérique.

7. Applicateur de matière de fermeture de plaie selon l'une des revendications précédentes, où l'élément de scellement comprend un doigt annulaire résiliant (84c) qui est positionné pour s'engager dans une paroi définissant le perçage du boîtier, le doigt annulaire réalisant un joint entre l'élément de scellement et le perçage définissant la paroi.

8. Applicateur de matière de fermeture de plaie selon l'une quelconque des revendications précédentes, où au moins un orifice d'admission précité est une pluralité d'orifices d'admission.

9. Applicateur de matière de fermeture de plaie selon l'une quelconque des revendications précédentes, comprenant en outre une barre d'amenée (36) supportée d'une manière mobile dans la portion de corps, la barre d'amenée étant fonctionnellement associée à la poignée mobile (22) de telle sorte que l'actionnement de la poignée mobile entraîne un mouvement distal de la barre d'amenée dans la portion de corps.

10. Applicateur de matière de fermeture de plaie selon la revendication 9, comprenant en outre un poussoir (62) supporté sur l'extrémité distale de la barre d'amenée dans la portion de corps, la poignée mobile étant actionnable pour amener la barre mobile et le poussoir distalement pour forcer une matière de fermeture de plaie de la portion de corps dans l'ensemble de pointe.

11. Applicateur de matière de fermeture de plaie selon la revendication 10, où le poussoir est réalisé intégralement avec la barre d'amenée.

12. Applicateur de matière de fermeture de plaie selon la revendication 9, 10 ou 11, où la barre d'amenée s'étend à travers l'ensemble de poignées et une plaque d'entraînement (32) positionnée dans l'ensemble de poignées, la poignée mobile étant actionnable pour amener la plaque d'entraînement en prise avec la barre d'amenée afin de déplacer la barre d'amenée distalement.

13. Applicateur de matière de fermeture de plaie selon la revendication 12, comprenant en outre une plaque de verrouillage (24) positionnée dans l'ensemble de poignées et ayant une fente (52) ménagée dans celle-ci, la barre d'amenée s'étendant à travers la fente, la plaque de verrouillage étant déplaçable d'une première position permettant à la barre d'amenée de passer à travers la fente à une seconde position de mise en prise avec la barre d'amenée pour empêcher un mouvement proximal de la barre d'amenée relativement à la plaque de verrouillage.

14. Applicateur de matière de fermeture de plaie selon la revendication 13, comprenant en outre un élément de sollicitation (56) positionné pour solliciter la plaque de verrouillage à la seconde position.

15. Applicateur de matière de fermeture de plaie selon l'une quelconque des revendications précédentes, comprenant la matière de fermeture de plaie.

16. Applicateur de matière de fermeture de plaie selon la revendication 15, où la matière de fermeture de plaie est un adhésif à base de cyano-acrylate.

17. Applicateur de matière de fermeture de plaie selon l'une quelconque des revendications précédentes, où l'ensemble de pointe comprend un élément de sollicitation (86) qui sollicite l'élément de scellement vers la position fermée.

18. Applicateur de matière de fermeture de plaie selon l'une quelconque des revendications précédentes, où le canal de corps défini un réservoir pour la matière de fermeture de plaie.
